(19)

Europäisches
Patentamt
European
Patent Office
Office européen
des brevets

(11) **EP 4 181 145 B1**

(12) **EUROPEAN PATENT SPECIFICATION**

(45) Date of publication and mention
of the grant of the patent:
**13.08.2025   Bulletin 2025/33**

(21) Application number: **22202493.7**

(22) Date of filing: **19.10.2022**

(51) International Patent Classification (IPC):
**G16B 15/30** (2019.01)   **G16B 20/30** (2019.01)
**G16B 40/20** (2019.01)   **G16B 40/30** (2019.01)

(52) Cooperative Patent Classification (CPC):
**G16B 15/30; G16B 20/30; G16B 40/20;
G16B 40/30**

(54) **METHOD AND SYSTEM FOR STRUCTURE-BASED DRUG DESIGN USING A MULTI-MODAL
DEEP LEARNING MODEL**

VERFAHREN UND SYSTEM FÜR STRUKTURBASIERTEN ARZNEIMITTELENTWURF UNTER
VERWENDUNG EINES MULTIMODALEN TIEFENLERNMODELLS

PROCÉDÉ ET SYSTÈME DE CONCEPTION DE MÉDICAMENT BASÉE SUR UNE STRUCTURE À
L'AIDE D'UN MODÈLE D'APPRENTISSAGE PROFOND MULTIMODAL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.11.2021   IN 202121052045**

(43) Date of publication of application:
**17.05.2023   Bulletin 2023/20**

(73) Proprietor: **Tata Consultancy Services Limited
Maharashtra (IN)**

(72) Inventors:
• **ROY, ARIJIT**
**500081 Hyderabad, Telangana (IN)**
• **SRINIVASAN, RAJGOPAL**
**500081 Hyderabad, Telangana (IN)**
• **VANGALA, SARVESWARARAO**
**500081 Hyderabad, Telangana (IN)**
• **KRISHNAN, SOWMYA RAMASWAMY**
**500081 Hyderabad, Telangana (IN)**
• **BUNG, NAVNEET**
**500081 Hyderabad, Telangana (IN)**
• **BULUSU, GOPALAKRISHNAN**
**500032 Hyderabad, Telangana (IN)**

(74) Representative: **Goddar, Heinz J.
Boehmert & Boehmert
Anwaltspartnerschaft mbB
Pettenkoferstrasse 22
80336 München (DE)**

(56) References cited:
• **JANNIS BORN ET AL: "PaccMann^RL:
Designing anticancer drugs from transcriptomic
data via reinforcement learning", ARXIV.ORG,
CORNELL UNIVERSITY LIBRARY, 201 OLIN
LIBRARY CORNELL UNIVERSITY ITHACA, NY
14853, 29 August 2019 (2019-08-29),
XP081632187**
• **XIONG ZHAOPING ET AL: "Pushing the
Boundaries of Molecular Representation for
Drug Discovery with the Graph Attention
Mechanism", JOURNAL OF MEDICINAL
CHEMISTRY, vol. 63, no. 16, 13 September 2019
(2019-09-13), US, pages 8749 - 8760,
XP093021828, ISSN: 0022-2623, DOI: 10.1021/
acs.jmedchem.9b00959**
• **YI ZHANG: "An In-depth Summary of Recent
Artificial Intelligence Applications in Drug
Design", ARXIV.ORG, CORNELL UNIVERSITY
LIBRARY, 201 OLIN LIBRARY CORNELL
UNIVERSITY ITHACA, NY 14853, 10 October 2021
(2021-10-10), XP091075766**
• **KRISHNAN SOWMYA RAMASWAMY ET AL: "De
Novo Structure-Based Drug Design Using Deep
Learning", vol. 62, no. 21, 18 November 2021
(2021-11-18), US, pages 5100 - 5109,
XP093034340, ISSN: 1549-9596, Retrieved from
the Internet <URL:https://pubs.acs.org/doi/pdf/
10.1021/acs.jcim.1c01319> [retrieved on
20230323], DOI: 10.1021/acs.jcim.1c01319**

EP 4 181 145 B1

**Description**

TECHNICAL FIELD

The disclosure herein generally relates to drug design, and, more particularly, to method and system for structure-based drug design using a multi-modal deep learning model.

BACKGROUND

Recent advancements and applications of deep learning methods in the field of drug design have led to a surge of interest and hope towards accelerating the drug design process. Primary efforts to cure vulnerable diseases involves identification of therapeutic molecules that modulates the activity of proteins responsible for such diseases. Various computational methods exist which improve the success rate of drug design process. However, most of these methods are ligand-based, where an initial target-specific ligand dataset is necessary to design potent molecules with optimized properties. Although there have been several attempts to develop alternative ways to design target-specific ligand datasets, but availability of such datasets remains a challenge while designing molecules against newer target proteins. One of the major challenge includes exploration of potentially unexplored chemical space which can be estimated using deep learning models. It is proven that deep learning methods not only explore the vast chemical space, but can also design new molecules on-the-fly with physicochemical property optimization towards the specific target protein. The time from early-stage drug design and optimization to experimental validation, has been drastically reduced with the help of such deep learning methods.

Drug design approaches against the specific target protein of interest can be broadly classified into ligand-based drug design and structure-based drug design. Majority of the deep learning-based drug design methods are ligand-based which use the existing knowledge of target-specific small molecules to design a set of more potent target-specific molecules with optimized properties through transfer learning and/or reinforcement learning. While ligand-based drug design methods have provided reliable results for several popular drug targets, their dependence on a dataset of existing target-specific ligands restricts their utility against newer target proteins and proteins with limited known ligand data.

In contrast, the structure-based drug design approach relies only on the structural features of the target protein to generate small molecules with complementary features which facilitate better binding. Traditionally structure-based drug design utilizes fragment growing and/or fragment linking methods. Few recent developments have also been applied on deep learning techniques to utilize the protein structure information for *de novo* design of new small molecules. Such methods can be broadly classified into two categories such as unsupervised method and semi-supervised method.

Among the structure-based drug design approaches using deep learning, one existing method utilizes graph representations of both the binding site and ligand, and in another existing method utilized a voxelated representation of the protein binding site to predict Simplified Molecular Input Line Entry System (SMILES) corresponding to the predicted complementary ligand shapes. Both the above existing methods are categorized as unsupervised binding site-based molecule generation approaches. On the other hand, another existing method utilized the entire protein sequence as input to the generative model. It is also to be noted that the application of reinforcement learning-based training for the generation of target-specific molecules uses complete protein sequence.

Document (JANNIS BORN ET AL: "PaccMann'RL: Designing anticancer drugs from transcriptomic data via reinforcement learning", ARXIV.ORG, CORNELL UNIVERSITY LIBRARY, 201 OLIN LIBRARY CORNELL UNIVERSITY ITHACA, NY 14853, 29 August 2019) discloses the first generative model capable of tailoring anticancer compounds for a specific biomolecular profile. Using a RL framework, the transcriptomic profiles of cancer cells are used as a context for the generation of candidate molecules. Our molecule generator combines two separately pretrained variational autoencoders (VAEs) - the first VAE encodes transcriptomic profiles into a smooth, latent space which in turn is used to condition a second VAE to generate novel molecular structures on the given transcriptomic profile. The generative process is optimized through PaccMann, a previously developed drug sensitivity prediction model to obtain effective anticancer compounds for the given context (i.e., transcriptomic profile). The molecule generation can be biased towards compounds with high predicted inhibitory effect against individual cell lines or specific cancer sites. The process is verified by investigating candidate drugs generated against specific cancer types and find the highest structural similarity to existing compounds with known efficacy against these cancer types.

SUMMARY

Embodiments of the present disclosure present technological improvements as solutions to one or more of the above-mentioned technical problems recognized by the inventors in conventional systems. For example, in one embodiment, a system for structure-based drug design using a multi-modal deep learning model is provided. The system includes processing an input having a target protein for drug design by using at least one of a multi-modal deep learning model comprising of a graph attention-based variational auto-encoder (GAT-VAE) module, a simplified molecular input line entry system based variational auto-encoder (SMILES-VAE) module, a conditional molecular generator, and a drug-target affinity (DTA) predictor module. The GAT-VAE module from the target protein obtains a latent vector of at least one of active site graph comprising of key amino acid residues. Here, the GAT-VAE module is pretrained to learn structure and type of interactions from amino acids lining the active site residues of the target protein. Further, the SMILES-VAE module obtains at least one latent vector from the target protein, wherein the SMILES-VAE module is pretrained to learn the grammar of small molecules. Then, the conditional molecular generator concatenates at least one latent vector of active site graph of the GAT-VAE module with the atleast one latent vector of the SMILES-VAE module to generate a set of molecules specific to the target protein. Further, iteratively performing by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset. Further, by using the conditional molecule generator at least one optimized molecule is designed with an affinity of the target protein greater than a pre-defined threshold score.

In another aspect, a method for structure-based drug design using a multi-modal deep learning model is provided. The method includes an input having a target protein for drug design by using at least one of a multi-modal deep learning model comprising of a graph attention-based variational auto-encoder (GAT-VAE) module, a simplified molecular input line entry system based variational auto-encoder (SMILES-VAE) module, a conditional molecular generator, and a drug-target affinity (DTA) predictor module. The GAT-VAE module from the target protein obtains a latent vector of at least one of active site graph comprising of key amino acid residues. Here, the GAT-VAE module is pretrained to learn structure and type of interactions from amino acids lining the active site residues of the target protein. Further, the SMILES-VAE module obtains at least one latent vector from the target protein, wherein the SMILES-VAE module is pretrained to learn the grammar of small molecules. Then, the conditional molecular generator concatenates at least one latent vector of active site graph of the GAT-VAE module with the atleast one latent vector of the SMILES-VAE module to generate a set of molecules specific to the target protein. Further, iteratively performing by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset. Further, by using the conditional molecule generator at least one optimized molecule is designed with an affinity of the target protein greater than a pre-defined threshold score.

In yet another aspect, anon-transitory computer readable medium provides one or more non-transitory machine-readable information storage mediums comprising one or more instructions, which when executed by one or more hardware processors perform actions includes an I/O interface and a memory coupled to the processor is capable of executing programmed instructions stored in the processor in the memory to method for structure-based drug design using a multi-modal deep learning model is provided. The method includes an input having a target protein for drug design by using at least one of a multi-modal deep learning model comprising of a graph attention-based variational auto-encoder (GAT-VAE) module, a simplified molecular input line entry system based variational auto-encoder (SMILES-VAE) module, a conditional molecular generator, and a drug-target affinity (DTA) predictor module. The GAT-VAE module from the target protein obtains a latent vector of at least one of active site graph comprising of key amino acid residues. Here, the GAT-VAE module is pretrained to learn structure and type of interactions from amino acids lining the active site residues of the target protein. Further, the SMILES-VAE module obtains at least one latent vector from the target protein, wherein the SMILES-VAE module is pretrained to learn the grammar of small molecules. Then, the conditional molecular generator concatenates at least one latent vector of active site graph of the GAT-VAE module with the atleast one latent vector of the SMILES-VAE module to generate a set of molecules specific to the target protein. Further, iteratively performing by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset. Further, by using the conditional molecule generator at least one optimized molecule is designed with an affinity of the target protein greater than a pre-defined threshold score.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for structure-based drug design using a multi-modal deep learning model, in accordance with some embodiments of the present disclosure.
FIG. 2 is a functional architecture of the multi-modal deep learning model for drug design using the system of FIG.1, in accordance with some embodiments of the present disclosure.

FIG. 3A and FIG.3B is a flow diagram of the structure-based drug design using a multi-modal deep learning model using the system of FIG.1, in accordance with some embodiments of the present disclosure.

FIG.4 illustrates predicted bioactivity distribution based on the drug target affinity module for the generated molecule of the target protein using the system of FIG.1, in accordance with some embodiments of the present disclosure and some of the generated molecules.

FIG.5 illustrates a heat map of attention coefficients for the active site residues of dopamine receptor protein using the system of FIG.1, in accordance with some embodiments of the present disclosure.

FIG.6 illustrates interactions between key active site residues identified from attention coefficients of a Dopamine receptor D2 (DRD2) and selected molecule using the system of FIG.1, in accordance with some embodiments of the present disclosure.

FIG.7 illustrates internal diversity of generated small molecules for Dopamine receptor (DRD2) and Janus Kinase 2 (JAK2) proteins using the system of FIG.1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible.

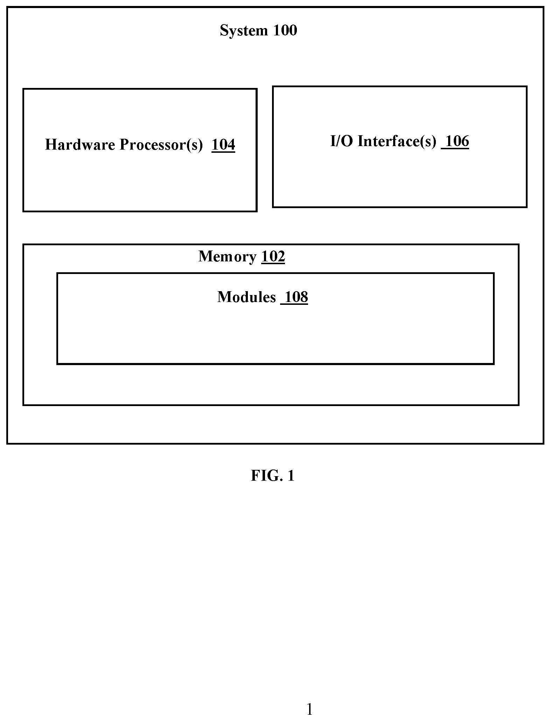

Embodiments herein provide a method and system for structure-based drug design using a multi-modal deep learning model. The multi-modal deep learning model improves diversity of small molecules. The semi-supervised multimodal deep learning model comprises of a graph attention-based variational auto-encoder (GAT-VAE) module, a simplified molecular input line entry system based variational auto-encoder (SMILES-VAE) module, a conditional molecular generator, and a drug-target affinity (DTA) predictor module. Initially, the method obtains a graph representation of one or more target protein binding sites from the GAT-VAE module and ligand representation from the SMILES-VAE module to design at least one optimized molecule for any target protein of known structure. The active site graph extracted from the GAT-VAE module and the small molecule from the SMILES-VAE module are fed as input to the conditional molecule generator, which is subject to a short re-training phase prior to optimization. Next, the DTA predictor module is used to formulate a reward function for target-specific bioactivity maximization, which is utilized as the objective to optimize the molecule generation process in a reinforcement learning framework. The designed molecules are evaluated and compared against experimentally known inhibitors such as a Janus Kinase 2 (JAK2) and a Dopamine receptor D2 (DRD2). The method of the present disclosure produces identical molecules when compared with the existing inhibitors, and while also retaining diversity. The set of generated molecules also have features of the existing inhibitors although the model had information about active site of the target proteins only. Finally, based on the GAT-VAE module, a set of key active site residues are identified which are responsible for favorable features of the generated new chemical entities. The disclosed system 100 is further explained with the method as described in conjunction with FIG.1 to FIG.7 below.

**Glossary:**

**Janus Kinase 2 (JAK2) protein** - An intracellular kinase protein involved in several immune response pathways specific to cancer and myeloproliferative disorders. This is one of the target proteins used for validating the proposed method.

**Dopamine receptor D2 (DRD2)** - A G-protein coupled receptor present in the brain and involved in regulation of dopamine release. This is one of the target proteins used for validating the proposed method.

**PDBbind** - An open source database containing experimentally determined protein-ligand complex structures and their binding affinities.

**sc-PDB** - An open source annotated database of druggable binding sites from the Protein Data Bank.

**UniProt-KB** - An open source database containing millions of protein sequences and several additional information regarding structure, function, mutations, disease associations and thereof for multiple organisms.

**CHEMBL database** - An open source database containing binding, functional and ADMET information for a large number of drug like bioactive compounds.

**AMSGrad optimizer** - Algorithm used to perform gradient descent optimization of the hyperparameters and weights of the neural network model during training.

**Astex diversity set** - Dataset of protein ligand complexes.

**Tanimoto coefficient (TC) -** A molecular similarity metric computed between binary representations of a pair of molecules.

**PharmaGist program -** An open source program to extract ligand-based pharmacophores.

**Pharmacophore based screening -** A method of identifying a subset of small molecules with pharmacophores similar to an input pharmacophore, based on three-dimensional alignments and scoring functions.

Referring now to the drawings, and more particularly to FIG. 1 through FIG.7, where similar reference characters denote corresponding features consistently throughout the figures, there are shown preferred embodiments and these embodiments are described in the context of the following exemplary system and/or method.

FIG. 1 illustrates an exemplary system for structure-based drug design using a multi-modal deep learning model, in accordance some embodiments of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

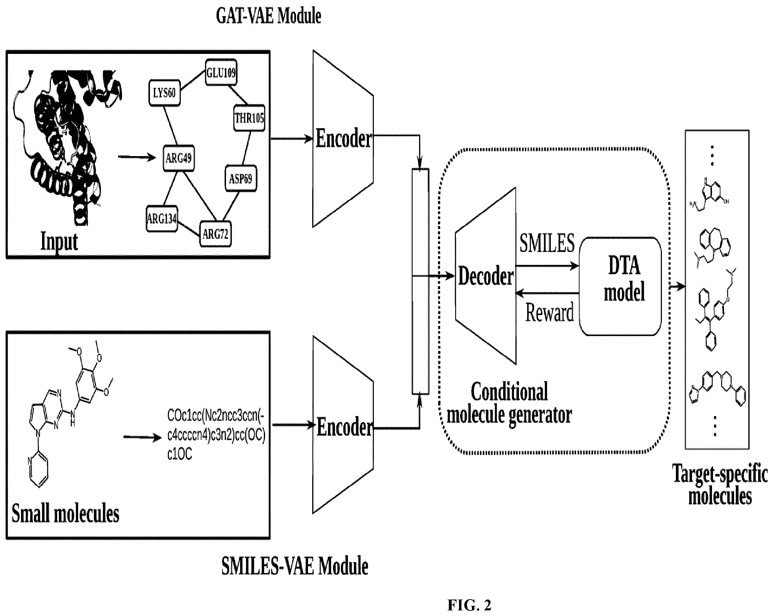

FIG. 2 is a functional architecture of the multi-modal deep learning model for drug design using the system of FIG. 1, in accordance with some embodiments of the present disclosure. FIG.2 includes a GAT-VAE module, a SMILES-VAE module, a conditional molecule generator, and a drug target affinity (DTA) predictor module. The GAT-VAE module is pre-trained to learn the active site graph and the SMILES-VAE module is pre-trained to learn the grammar of small molecules. The conditional molecule generator is a combination of the GAT-VAE module and the SMILES-VAE module. The DTA predictor module predicts bioactivity of generated small molecules and then the conditional molecule generator is fine-tuned using the DTA module (critic) in a reinforcement learning framework.

The GAT-VAE module is a neural network for embedding the active site graphs, wherein the GAT-VAE module comprises of an encoder and a decoder. The adjacency matrix ($A$) and node feature vector ($X$) of the graph are considered as input to the encoder. After extensive hyperparameter tuning, the encoder consisted of 5 parallel attention heads with a hidden size of 128 dimensions each. This is followed by a single head GAT layer for aggregation of the output from the 5 parallel heads. The aggregated output node features are passed through two parallel single head GAT layers to obtain mean and log variance vectors which are subject to reparameter-ization to obtain the latent vector. A dropout rate of 0.2 prevents over-fitting of the model. Finally, the encoder returns a 256-dimensional latent vector ($z$) of the input active site graph. The decoder is a standard inner-product decoder which utilizes the latent vector to reconstruct the adjacency matrix of the input active site graph. The GAT-VAE module is trained to minimize a joint loss function composed of the binary cross entropy loss for adjacency matrix reconstruction, and the Kullback-Leibler divergence (KLD) loss for enforcing the latent variables to follow the gaussian distribution. Adam optimizer is used to train the module with an initial learning rate of 0.001. The training dataset is splitted into minibatches of 256 graphs each. The module is trained for about 100 epochs in a Tesla®V 100 GPU where all implementations were performed using PyTorch optimized tensor library.

The SMILES-VAE module captures the grammar of small molecules by a recurrent neural network (RNN). Next, variational autoencoders (VAE) are used to learn both the active site and small molecule embeddings. The active site graph with one embedding is utilized to condition the generative process. The reinforcement learning (RL) framework is used with the conditional molecule generator ( combination of the pre-trained GAT-VAE and the SMILES-VAE) as the agent, and the pre-trained DTA predictor module as the critic.

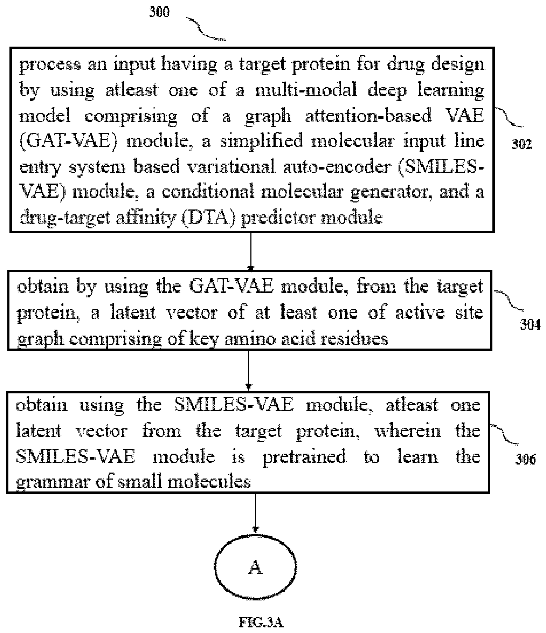

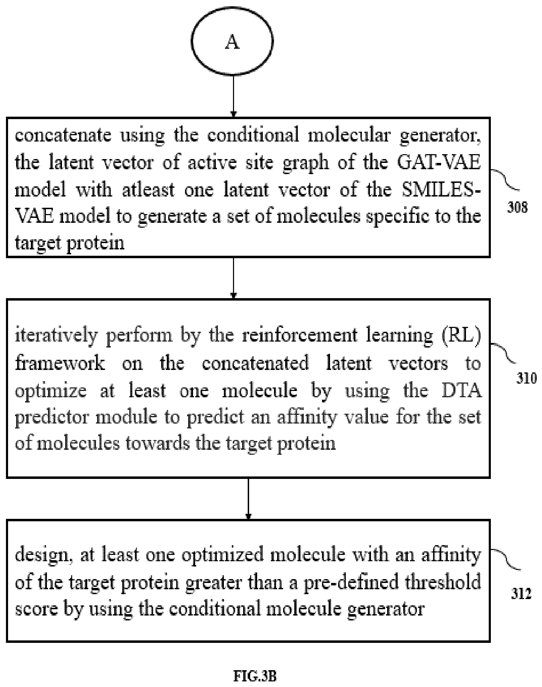

FIG. 3A and FIG.3B is a flow diagram of the structure-based drug design using a multi-modal deep learning model using the system of FIG.1, in accordance with some embodiments of the present disclosure. In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the alarm identification system 100 as depicted in FIG.3A and FIG.3B. Although process steps, method steps, techniques or the like may be described in a sequential order, such processes, methods and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 302 of the method 300 the one or more hardware processors 104 process an input having a target protein for drug design by using at least one of a multi-modal deep learning model comprising of a graph attention-based VAE (GAT-VAE) module, a simplified molecular input line entry system based variational auto-encoder (SMILES-VAE) module, a conditional molecular generator, and a drug-target affinity (DTA) predictor module. Referring now to an example where the method processes the input having a target protein received from one or more external sources for designing new drug structures. The components of the multi-modal deep learning processes the target protein to design at least one drug molecule. Further, processing steps are explained with the method in sequence with the embodiments of the present disclosure.

At step 304 of the method 300 the one or more hardware processors 104 obtain by using the GAT-VAE module, from the target protein a latent vector of at least one of active site graph comprising of key amino acid residues, wherein the GAT-VAE module is pretrained to learn structure and type of interactions from amino acids lining the active site residues of the target protein. Referring now to FIG.2, the active site of the target protein is composed of key amino acid residues which interact with at least one small molecule (ligand). The small molecules are capable of binding with the protein that are unknown a priori, and the representation of the active site is ligand-agnostic. At this stage, the model aims to learn the structure and the type of interactions between the different amino acid residues in the active site. The active site is represented as a graph where nodes represent amino acids, and edges represent the interaction between two amino acids within a pre-defined distance cut-off from each other (FIG.2). Further, the constructed active site graph resembles an atom pair contact network. The nodes of the active site graph are featured by classifying the amino acids into at least one of 7 classes based on volume and dipole moment, along with their ability to act as hydrogen bond donor and hydrogen bond acceptor, leading to a total of 9 node features. The unweighted adjacency matrix and the one-hot encoded node feature vector of the active site graph is provided as input to the GAT-VAE module.

In one embodiment, the GAT-VAE module is pretrained using the dataset of active sites collated from known databases such as PDBbind and sc-PDB. The PDBbind database comprises of a general set and a refined set of protein-ligand complexes. The general set consists of 12,800 complexes and the refined set consists of 4,852 complexes. Due to the observed redundancy of the proteins represented in the PDBbind database, the UniProt-KB IDs of the proteins were used to identify redundant proteins and retain only a unique representative of the protein. The sc-PDB database consists of 17,594 complexes, which were compared to both PDBbind general and refined set complexes. After removing overlapping and redundant complexes and active sites with non-standard amino acids, the PDBbind general set, refined set and sc-PDB database were combined to obtain a total of 5,981 active sites for training the GAT-VAE module. All pre-processing steps were done through in-house perl and python scripts.

At step 306 of the method 300 the one or more hardware processors 104 obtain by using the SMILES-VAE module, at least one latent vector from the target protein, wherein the SMILES-VAE module is pretrained to learn the grammar of small molecules. The SMILES-VAE module is pretrained using the dataset of drug-like small molecules in SMILES format was obtained from the known ChEMBL database. The SMILES dataset are pre-processed using the procedure RDKit library. Dataset of ~1.6 million drug like small molecules in simplified molecular line entry system (SMILES) format are used for pre-training the generative module. The deep neural network architecture of the SMILES-VAE module consists of an encoder and a decoder (FIG.2). Both encoder and decoder consist of two layers of 1024 bidirectional gated recurrent units (GRU) as the internal memory, augmented with a stack acting as the dynamic external memory. The stack had a width of 256 units and a depth of 100 units. An embedding layer and a dense layer with log softmax activation were used to pass the input to the encoder and retrieve the output from the decoder respectively.

The SMILES-VAE module training was performed using mini-batch gradient descent with AMSGrad optimizer (a variant of ADAM optimizer) with batch size and initial learning rate are set to 256 and 0.0005, respectively. A dropout rate of 0.2 prevents over-fitting of the module. Learning rate decay and gradient clipping were used to prevent vanishing and exploding gradients. The module is trained with 100 epochs on a Tesla® V100 GPU and the weights from the trained model were used for the downstream tasks in the pipeline and all implementations were performed using the PyTorch library.

At step 308 of the method 300 the one or more hardware processors 104 concatenate by using the conditional molecular generator, the latent vector of active site graph of the GAT-VAE module with at least one latent vector of the SMILES-VAE module to generate a set of molecules specific to the target protein. Here, the set of molecules are generated from the concatenated latent vectors. Pre-trained GAT-VAE module and the SMILES-VAE module are combined (FIG.2) to condition the molecule generation process to generate molecules specific to the target protein. To condition the SMILES-VAE module, the latent vector of the input graph ($z_g$) from the GAT-VAE encoder are concatenated with the latent vector corresponding to a primer string ($z_s$) from the SMILES-VAE encoder, to form the combined latent vector ($z$). The primer string usually corresponds to the Start-Of-Sequence (SOS) character which can also be replaced by a scaffold or molecular group that needs to be present in the generated small molecule. The conditional molecule generator is pre-trained with one or more unique active site small molecule pairs from the PDBbind dataset (general set and refined set). This short pre-training enabled the SMILES-VAE decoder to learn to decode the combined latent vector ($z$) with more chemical validity in comparison to the module without pre-training. The pre-training of conditional molecule generator is done with AMSGrad optimizer for 50 epochs, with an initial learning rate of 0.0005 and a batch size of 256. The cross-entropy loss for the reconstruction of the small molecule corresponding to the active site was used for training.

At step 310 of the method 300 the one or more hardware processors 104 iteratively perform by the reinforcement learning (RL) framework on the concatenated latent vectors to optimize at least one small molecule by using the drug-target affinity (DTA) predictor module to predict an affinity value for the set of small molecules towards the target protein, wherein the DTA predictor module is pretrained using a drug protein dataset. The drug-target affinity (DTA) predictor module is pretrained using the training dataset of active small molecules against various target proteins. This training dataset includes small molecules spanning both high and low ends of the bioactivity spectrum to improve the ability of a predictive model to accurately predict a quantity of interest for an external dataset, which was not shown to the model during training and validation of the DTA predictor module for new small molecules. All active site small molecule pairs from the PDBbind general set and refined set with experimentally determined a half maximal inhibitory concentration ($pIC_{50}$), an inhibitory constant ($K_i$) and a dissociation constant ($K_d$) values were collected amounting to a set of 9,584 unique datapoints. All the datapoints were scaled to their corresponding molar concentrations and converted to log scale.

The drug-target affinity (DTA) predictor module measures the affinity of the generated small molecules towards the target protein (FIG.2). The DTA predictor module is designed based on extended connectivity interaction fingerprints (ECIF). ECIF is used to represent a protein-ligand complex as a vector of 1540 integer-valued features, where each position corresponds to the count of a pair-wise combination of protein-ligand atom types with preserved directionality. Therefore, the protein-ligand complexes from the general and refined set of PDBbind dataset were used to train the DTA module with ECIF and 170 RDKit chemical descriptors as inputs. The DTA module is a gradient-boosted tree (GBT) built using the hyperparameters described in the ECIF: 20,000 boosting stages, maximum depth of 8, a learning rate of 0.005, least squares regression as the loss function, a fraction of samples to fit the individual learners of 0.7, and "sqrt" as the fraction of features to look at for the best split. Pearson correlation coefficient ($R_p$) and the root mean square error (RMSE) were used as the metrics to assess the module performance. The module was validated on the PDBbind core set and tested on the Astex diversity set.

At step 312 of the method 300 the one or more hardware processors 104 design by using the conditional molecule generator at least one optimized molecule with an affinity of the target protein is greater than a pre-defined threshold score. The set of molecules are associated with a binding affinity which is greater than or equal to the predefined threshold score. Here, at least one small molecule for the target protein is designed based on applying one or more physio chemical properties and toxicity filters on each target protein specific to the molecule from the set of molecules to obtain a reduced set of target specific molecules. Further, the reinforcement learning framework combines the conditional molecule generator (agent) and the DTA module (critic) to design new small molecules for any given target protein (FIG.2). The generation and optimization cycle starts by providing the active site graph of the target protein as input to the conditional molecule generator. For every iteration, 50 molecules were sampled using the conditional molecule generator for an input active site graph and passed to the DTA predictor module (critic) for evaluation. The number of molecules to be sampled per iteration was chosen based on extensive hyperparameter tuning. The DTA predictor module predicts the ($pIC_{50}$) value of each generated molecule with respect to the target active site. The protein-ligand complex required to compute the ECIF fingerprints for the generated small molecules was obtained through on-the-fly docking of molecules to the target active site. This predicted ($pIC_{50}$) value is used to calculate the reward/penalty using a reward function as described below in equation 1,

$$r(x) = \exp\left(\frac{x}{3.0}\right) \qquad \text{------------ equation 1}$$

Here, x refers to the predicted ($pIC_{50}$) value of the generated molecule. The reward or penalty from the reward function is used in a regularized loss function which prevents "catastrophic forgetting" of the features learnt by the module. The generation and optimization cycle continues until the bioactivity distribution for the generated small molecules is well optimized. Termination of RL training process is target protein-dependent, and multiple criteria are considered including, validity of the generated molecules, presence of duplicates, extent of bioactivity optimization, and rate of reproduction of molecules from the training ChEMBL database. Such criteria shall not be construed as limiting the scope of the present disclosure. In an embodiment of the present disclosure, only one criterion can be considered, or a combination of criteria may be considered. Such criteria combination and selection may be either performed by the system 100 or via one or more inputs from user(s), in one example embodiment.

In one embodiment, the designed molecule is *in silico* validated using the dataset of known inhibitors to understand the quality of the generated molecules. The generated molecules were compared with small molecules specific to two target proteins such as Janus kinase 2 (JAK2) and Dopamine receptor D2 (DRD2). The datasets of all known inhibitors of the JAK2 and the DRD2 along with their experimentally determined ($pIC_{50}$) values, were collected from the ChEMBL database. These datasets were pre-processed following the procedure from existing techniques after which, the JAK2 and the DRD2 validation datasets contained 1,103 and 4,221 compounds, respectively.

Once the RL is trained, a set of 10,000 small molecules were generated with predicted bioactivity values for each of the target proteins. The quality of the generated small molecules are validated by measuring the similarity of the generated small molecules to known ligands of the target protein (validation dataset, mentioned earlier), based on a known metric, Tanimoto coefficient (TC). The similarity of various physicochemical property distributions of generated small molecules and known ligands were also compared. In terms of the substructure similarity, two different analyses such as (a) a fragment distribution and (b) a pharmacophore-based screening were performed. The pharmacophore-based screening explains geometric arrangement of atoms or functional groups of the generated molecules, that are essential for target inhibition. While Tanimoto coefficient enabled identification of generated molecules that are similar to the validation dataset, the latter two substructure analysis methods helped to identify molecules with spatial features similar to the validation dataset, albeit with diversity.

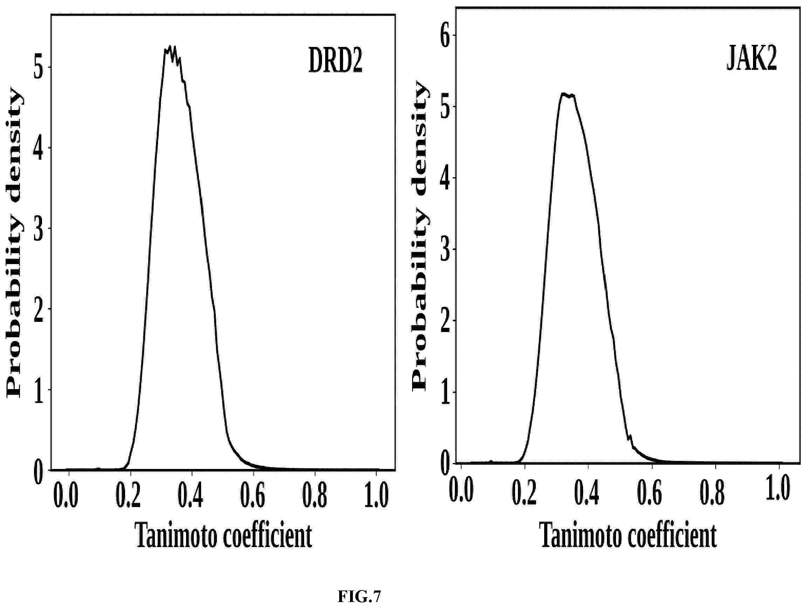

Internal diversity of generated molecules (FIG.7) is verified by making all pairwise comparisons within the molecules in the dataset by looking at the distribution of similarity metrics from the comparison. Using ECFP4 fingerprints as the molecular representation and the Tanimoto coefficient as the similarity metric, the internal diversity of the JAK2- and DRD2-specific generated molecules were verified. In both cases, the mean of the similarity distribution was below 0.5 indicating that on average, most of the molecule pairs within the generated dataset are less than 50% similar to each other. This observation shows that the generated molecules are highly diverse and the internal diversity of the dataset is very high. Hence, the conditional molecule generator is capable of generating highly diverse dataset of molecules for any given target active site of interest, as shown here for the JAK2 and the DRD2 proteins.

The PharmaGist program was used for ligand-based pharmacophore analysis. To extract the ligand-based pharmacophores, the existing inhibitors of the target protein were clustered using Butina clustering in RDKit with Tanimoto coefficient as the distance metric, and 0.4 as the distance cutoff. Since, PharmaGist program can take only 32 molecules as input, clustering was used to narrow down the size of the validation dataset. From the clustering results, clusters with at least 10 molecules were chosen and the representative molecules of such clusters were collected. A random set of 32 molecules from this list was used as input to the PharmaGist program. The top 2 composite ligand-based pharmacophores were chosen based on coverage of the active site, and ability to represent at least 95% of the molecules present in the validation dataset. They were used to screen the database of generated small molecules specific to the target protein of interest.

In one embodiment performance of the pretrained module on the ChEMBL dataset was evaluated using the GuacaMol distribution learning benchmark (v0.5.3). The metrics of the benchmark include: validity, uniqueness, newly generated molecules, Kullback-Leibler divergence (KLD) and Fréchet ChemNet distance (FCD). The module is 93.22% accurate in decoding SMILES strings from their latent representations, with 99% uniqueness and 96% newly generated molecules among the sampled small molecules. In comparison to the baseline VAE model highlighted in the GuacaMol benchmark, the pre-trained module int the validity metric (Table 1). Table 1 depicts comparison between the benchmark metrics of the baseline VAE model from the GuacaMol benchmark and the module in the present disclosure.

**Table 1. Benchmarking results**

| Model | Validity | Uniqueness | Newly generated molecules | Fréchet ChemNet distance (FCD) | Kullback-Leibler divergence |
|---|---|---|---|---|---|
| GuacaMol baseline VAE model (Brown et al., 2019) | 0.870 | 0.999 | 0.974 | 0.863 | 0.982 |
| SMILES-VAE model | 0.932 | 0.999 | 0.969 | 0.847 | 0.981 |

The two different GAT-VAE module were trained on active site graph datasets created with two distance cut-offs for edge definition such as a) model 1 with 4 Å and b) model 2 with 5 Å. The models were trained on the task of reconstructing the adjacency matrix from the latent embedding of the active site graph. The region of curve (ROC) score for the model 1 and 2 was 0.89 and 0.84, respectively. Based on the validation of ROC scores, edge permutation tests, and cues from the literature on protein interaction networks, model 1 was chosen for further analyses. The drug-target affinity predictor model was validated with the PDBbind core set and tested with the Astex diversity set. Pearson correlation coefficient ($R_p$) and the root mean square error (RMSE) were used as the evaluation metrics for the model. The Pearson correlation coefficient ($R_p$) for PDBbind core set and Astex diversity set was 0.86 (RMSE = 1.16) and 0.57 (RMSE = 1.51), respectively. It is notable that the DTA predictor module of the present disclosure performs better (in terms of ($R_p$)) than the existing (or conventional) DTA predictor module for the Astex diversity set.

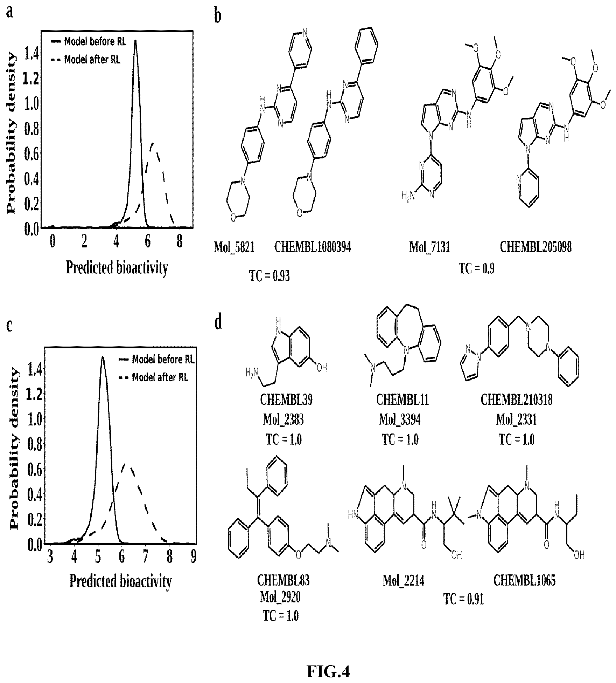

FIG.4 illustrates predicted bioactivity distribution based on the drug target affinity model for the generated molecule of the target protein using the system of FIG. 1, in accordance with some embodiments of the present disclosure. The pre-trained conditional molecule generator are used as the agent in a reinforcement learning framework, along with the DTA predictor model as the critic, to generate new small molecules specific to the target proteins. Two well-studied proteins such as the JAK2 (PDB ID: 3UGC), an intracellular protein belonging to the ubiquitous kinase family, and DRD2 (PDB ID: 6CM4), a G-protein coupled receptor (GPCR) present in the central nervous system, were chosen. The large number of available inhibitors for the JAK2 and the DRD2 provides an opportunity for *in silico* validation of the proposed method.

For each target protein, the conditional molecule generator is trained individually with corresponding binding site graph until a sufficient shift in the distribution of bioactivity values (predicted by DTA predictor module) are observed. The final bioactivity distributions obtained after the training process are shown below (FIG.4). After the reinforcement learning training process, the resultant target-specific conditional molecule generator module is used to sample 10,000 small molecules. Molecules that were chemically invalid were removed, and the rest of the molecules were canonicalized before further analysis. In both the cases, the model after reinforcement learning could generate an average of 90% valid molecules indicating that the model has overcome catastrophic forgetting effectively.

In another embodiment, analysis of the generated small molecules were evaluated with *in silico* validation by comparing existing inhibitors of the target proteins with the generated molecules. The similarity of the generated molecules was checked with the Tanimoto coefficient and the pharmacophoric distributions.

Similarity of generated molecules based on Tanimoto coefficient: First the similarity of the generated small molecules to a target-specific dataset of molecules was computed using the Tanimoto coefficient (TC) with ECFP4 fingerprints45 as input representations. A TC cut-off of 0.75 was used to identify the subset of generated molecules which have high similarity to existing molecules for a target protein. Based on the comparison it was identified that 30 and 80 generated small molecules met the TC cut-off requirement for the JAK2 and the DRD2 proteins, respectively (FIG.4). Further, generated small molecules were found to be identical with the existing DRD2 inhibitors (TC = 1.0), showcasing the ability of the conditional generator module to reproduce existing inhibitors for a target protein. One of the limitations of TC based scoring of ECFP4 fingerprints is that it does not consider the feature similarities among functional groups present in the two molecules. This leads to identification of only a subset of generated molecules whose structure is extremely similar to the existing inhibitors, but misses the other diverse molecules, which can still possess the required functional groups, or the pharmacophore features necessary for biological response.

**Similarity of the generated molecules based on ligand-based pharmacophores:** The ligand-based pharmacophores extracted using the PharmaGist program, were used to screen the generated small molecules and identify molecules with high feature overlap score. Such molecules can be considered as efficient inhibitors despite their lower ECFP4-based Tanimoto similarity compared to existing inhibitors. The small molecule considered as a hit, if the feature overlap score of the molecule with the target pharmacophore was at least half of the maximum feature overlap score. The hits among the generated small molecules were filtered for both the JAK2 and the DRD2 proteins. The results of the pharmacophore-based screening are summarized in Table 2. Based on the results it is observed that, 87% of the JAK2-specific generated molecules, and 84% of the DRD2-specific generated molecules could be covered by the target-specific ligand-based pharmacophores of the respective proteins.

**Results from the pharmacophore-based screening of generated small molecules for JAK2 and DRD2 proteins (Table 2):** The percentage of hits, number of molecules screened by either pharmacophores, and molecules which are not screened by both the pharmacophores are provided.

Table 2 - Results from the pharmacophore-based screening of generated small molecules for JAK2 and DRD2 proteins

| Protein | Pharmacophore | Hits* (%) | Screened count** | Not screened count | Screened by the other pharmacophore | Not screened by both pharmacophores |
|---|---|---|---|---|---|---|
| DRD2 validation set | Pharmacophore 1 | 97.26 | 4162 | 44 | 39 | 5 |
| DRD2 validation set | Pharmacophore 2 | 97.95 | 4158 | 48 | 43 | |
| DRD2 generated set | Pharmacophore 1 | 84.63 | 8475 | 761 | 329 | 432 |
| DRD2 generated set | Pharmacophore 2 | 85.09 | 8399 | 837 | 405 | |
| JAK2 validation set | Pharmacophore 1 | 99.72 | 1103 | 0 | 0 | 0 |
| JAK2 validation set | Pharmacophore 2 | 100 | 1103 | 0 | 0 | |
| JAK2 generated set | Pharmacophore 1 | 87.45 | 8577 | 27 | 15 | 12 |
| JAK2 generated set | Pharmacophore 2 | 94.76 | 8588 | 16 | 4 | |

*- Percentage of molecules with at least half the maximum overlap score are considered as hits;

** - Any molecule with a positive overlap score is considered as a screened molecule.

Similar to the DRD2, two pharmacophores were identified based on the coverage of the active site of JAK2. It is clear from the pharmacophore-based screening results that the generated small molecules captures the key pharmacophore features of the target active site. To further confirm the pharmacophore-level similarity of the generated small molecules to the existing inhibitors, two pharmacophore fingerprints (ErGFP and PharmacoPFP) were calculated. The pharmacophore fingerprints of generated small molecules and existing inhibitors were compared using cosine similarity. The distribution of the cosine similarity values from all pairwise comparisons shows that above 90% of the generated small molecules have high pharmacophore-level similarity (cosine similarity above 0.8) to existing inhibitors.

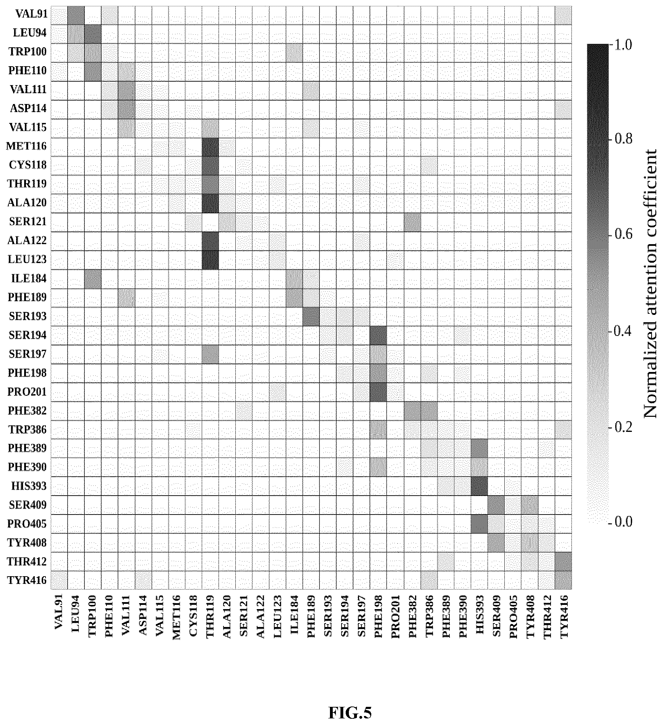

FIG.5 illustrates a heat map of attention coefficients for the active site residues of dopamine receptor protein using the system of FIG. 1, in accordance with some embodiments of the present disclosure. The attention coefficients from the GAT-VAE module were analyzed for each residue (node) and its neighborhood in the active site graph. In essence, the attention coefficients define an interaction probability distribution over each node and its neighbors in the graph. By analyzing the attention coefficients for each node, the residue pairs which are frequently given more attention by the model can be identified, and the biological significance behind the latent representations are learned by the GAT-VAE module can be elucidated. By considering the attention coefficients of a node's neighborhood as probabilities, the information content of the coefficients can be calculated using Shannon's entropy. Skewness in the entropy distribution compared to the uniform distribution indicates that, the module has learned to give importance to a subset of node neighbors, rather than providing equal weight for all the neighbors of a given node. The key residues and interactions at the DRD2 binding site identified from the attention coefficient heatmap are shown in (FIG.5). Residue pairs with attention coefficient above 0.5 were considered important. For the binding site of the DRD2 protein (PDB ID: 6CM4), only 17 of the 149 interactions had attention coefficient ($\alpha_{ij}$) above 0.50 from the GAT-VAE module. The 8 active site residues (Leu94, Trp100, Asp114, Thr119, Ile184, Phe198, His393 and Tyr416) are involved with attention coefficients above 0.5. These 8 active site residues are known to interact with various highly selective DRD2 inhibitors reported in literature.

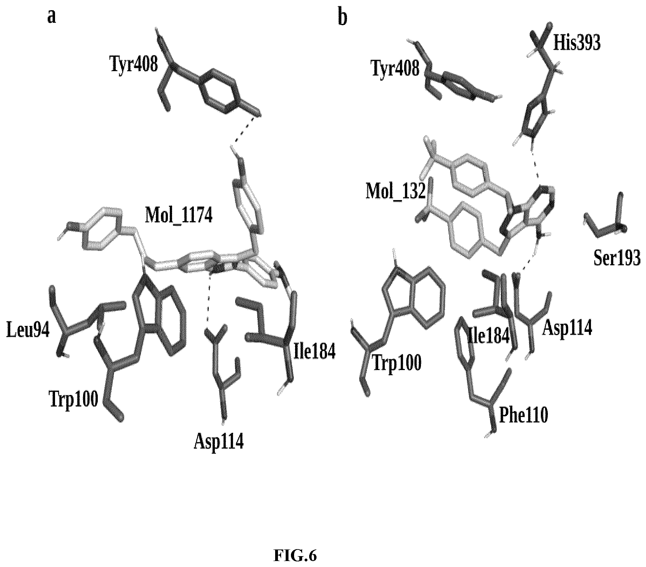

FIG.6 illustrates interactions between key active site residues identified from attention coefficients of a Dopamine receptor D2 (DRD2) and selected molecule using the system of FIG.1, in accordance with some embodiments of the present disclosure. The residues found to interact with generated molecules. The interactions between two representative generated molecules with these key active site residues are shown in FIG.6. The active site of DRD2 is partially hydrophobic (Leu94, Trp100, Ile184, Phe110). These residues form hydrophobic interactions with the generated molecules. While on the other side it is lined with polar and charged residues (Asp114, Thr119, Ser193, His393 and Tyr408), which form hydrogen bond interactions with the generated molecules. Additionally, Tyr408 can also form stacking interactions with the generated molecules.

Three stabilizing interactions among DRD2 active site residues - His393 and Tyr408 ($\alpha_{ij}$ = 0.6), Ile184 and Trp100 ($\alpha_{ij}$ = 0.5), Trp100 and Leu94 ($\alpha_{ij}$ = 0.6) are also reported previously in literature. It is interesting to note that, mutation studies have proven the importance of interactions between Leu94, Trp100 and Ile184 in stabilizing the protein-ligand complex, and dissociation of the ligand from the binding site. Also, an inter-helical hydrogen bond between His393 and Tyr408 has been shown to stabilize the outward movement of the transmembrane helix VI in DRD2, which controls the switch between active and inactive states of the protein. The presence of a secondary amine group in the vicinity of the active site residue Asp114, helps in hydrogen bond formation (FIG.6). According to the previous literature, the Asp114 interaction is responsible to anchor the small molecules in the active site cavity. Overall, the residue pairs with higher attention coefficients were found to provide stability to the generated molecules and their role are also known from previous literature. Deep learning models are often criticized as black boxes, but the method as described herein by the system of the present disclosure explains the importance of active site residues. Possibly, these residues play a role in molecule generation, which can be explained from the complementarity of interactions with the generated molecules in case of DRD2 protein.

The key binding site residues of the JAK2 active site govern the interactions with generated small molecules and identified from the attention coefficient heatmap (FIG.5). These observations show that the GAT-VAE module can distinguish key binding site residues and interactions from the rest and incorporate that information in the latent vector of the active site graph by learning sharper attention coefficients. This also shows the usefulness of attention-based methods in enabling a better understanding of the features learned by the deep neural network model from a biological standpoint.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

The embodiments of present disclosure herein addresses the problem of structure-based molecule design. The embodiment, thus provides method and system for designing molecule for target protein using a multi-modal deep learning model. Moreover, the embodiments herein further provides structure-based drug design where the conditional molecule generator learn from the combined latent vectors of the existing two-dimensional representation of the protein active sites and SMILES-based (one dimensional) representation of molecules and can design new and diverse molecules according to the structure of a target protein. The final set of designed molecules has high binding affinity towards the target protein.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.  A processor implemented method (300) for structure-based drug design using a multi-modal deep learning model, comprising:

    processing (302), via one or more hardware processors (104), an input having a target protein for drug design by using:

    (a) a multi-modal deep learning model, wherein the multi-modal learning model comprises a graph attention-based variational auto-encoder (GAT-VAE) module,
    (b) a simplified molecular input line entry system based variational auto-encoder (SMILES-VAE) module,
    (c) a conditional molecular generator, and
    (d) a drug-target affinity (DTA) predictor module;
    obtaining (304), via the one or more hardware processors (104), by using the GAT-VAE module from the target protein, a latent vector of at least one of active site graph comprising of key amino acid residues, wherein the GAT-VAE module is pretrained to learn structure and type of interactions from amino acids lining the active site residues of the target protein;
    obtaining (306), via the one or more hardware processors(104), by using the SMILES-VAE module, at least one latent vector from the target protein, wherein the SMILES-VAE module is pretrained to learn the grammar of small molecules;

concatenating (308), via the one or more hardware processors (104), by using the conditional molecular generator, at least one latent vector of active site graph of the GAT-VAE module with the atleast one latent vector of the SMILES-VAE module to generate a set of molecules specific to the target protein;

iteratively performing (310), via the one or more hardware processors (104), by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset; and

designing (312), via the one or more hardware processors (104), by using the conditional molecule generator, at least one optimized molecule with an affinity of the target protein is greater than a pre-defined threshold score.

2. The processor implemented method (300) as claimed in claim 1, wherein the conditional molecular generator concatenates atleast one latent vector of the input active site graph ($z_g$) from an encoder of the GAT-VAE module with atleast one latent vector corresponding to a primer string ($z_s$) from the encoder of the SMILES-VAE module to form a combined latent vector ($z$).

3. The processor implemented method (300) as claimed in claim 1, wherein the conditional molecular generator is pretrained with training datasets of one or more active site graphs and one or more molecules.

4. The processor implemented method (300) as claimed in claim 1, wherein designing at least one small molecule for the target protein is based on applying one or more physio chemical properties and toxicity filters on each target protein specific to the molecule from the set of molecules to obtain a reduced set of target specific molecules.

5. The processor implemented method (300) as claimed in claim 1, wherein the set of molecules are associated with a binding affinity which is greater than or equal to the predefined threshold score.

6. A system (100) for structure-based drug design using a multi-modal deep learning model, comprising:

a memory (102) storing instructions;

one or more communication interfaces (106); and

one or more hardware processors (104) coupled to the memory (102) via the one or more communication interfaces (106), wherein the one or more hardware processors (104) are configured by the instructions to:

process, an input having a target protein for drug design by using:

(a) a multi-modal deep learning model, wherein the multi-modal learning model comprises a graph attention-based variational auto-encoder (GAT-VAE) module,

(b) a simplified molecular input line entry system based variational auto-encoder (SMILES-VAE) module,

(c) a conditional molecular generator, and

(d) a drug-target affinity (DTA) predictor module;

obtain, by using the GAT-VAE module from the target protein, a latent vector of at least one of active site graph comprising of key amino acid residues, wherein the GAT-VAE module is pretrained to learn structure and type of interactions from amino acids lining the active site residues of the target protein;

obtain, by using the SMILES-VAE module, at least one latent vector from the target protein, wherein the SMILES-VAE module is pretrained to learn the grammar of small molecules;

concatenate, by using the conditional molecular generator, at least one latent vector of active site graph of the GAT-VAE module with the atleast one latent vector of the SMILES-VAE module to generate a set of molecules specific to the target protein;

iteratively perform, by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset; and

design, by using the conditional molecule generator at least one optimized molecule with an affinity of the target protein is greater than a pre-defined threshold score.

7. The system (100) as claimed in claim 6, wherein the conditional molecular generator concatenates atleast one latent vector of the input active site graph ($z_g$) from an encoder of the GAT-VAE module with atleast one latent vector corresponding to a primer string ($z_s$) from the encoder of the SMILES-VAE module to form a combined latent vector ($z$).

8. The system (100) as claimed in claim 6, wherein the conditional molecular generator is pretrained with training datasets of one or more active site graphs and one or more molecules.

9. The system (100) as claimed in claim 6, wherein designing at least one small molecule for the target protein is based on applying one or more physio chemical properties and toxicity filters on each target protein specific to the molecule from the set of molecules to obtain a reduced set of target specific molecules.

10. The system (300) as claimed in claim 6, wherein the set of molecules are associated with a binding affinity which is greater than or equal to the predefined threshold score.

11. One or more non-transitory machine-readable information storage mediums comprising one or more instructions which when executed by one or more hardware processors cause:

   processing, an input having a target protein for drug design by using:

   (a) a multi-modal deep learning model comprising of a graph attention-based variational auto-encoder (GAT-VAE) module,
   (b) a simplified molecular input line entry system based variational auto-encoder (SMILES-VAE) module,
   (c) a conditional molecular generator, and
   (d) a drug-target affinity (DTA) predictor module;

   obtaining, by using the GAT-VAE module from the target protein, a latent vector of at least one of active site graph comprising of key amino acid residues, wherein the GAT-VAE module is pretrained to learn structure and type of interactions from amino acids lining the active site residues of the target protein;
   obtaining, by using the SMILES-VAE module, at least one latent vector from the target protein, wherein the SMILES-VAE module is pretrained to learn the grammar of small molecules;
   concatenating, by using the conditional molecular generator, at least one latent vector of active site graph of the GAT-VAE module with the atleast one latent vector of the SMILES-VAE module to generate a set of molecules specific to the target protein;
   iteratively performing, by a reinforcement learning (RL) framework on the concatenated latent vector to optimize at least one molecule by using the drug-target affinity (DTA) predictor module to predict an affinity value for the set of molecules towards the target protein, wherein the DTA predictor module is pretrained using a drug protein dataset; and
   designing, by using the conditional molecule generator, at least one optimized molecule with an affinity of the target protein is greater than a pre-defined threshold score.

12. The one or more non-transitory machine-readable information storage mediums of claim 11, wherein the conditional molecular generator concatenates atleast one latent vector of the input active site graph ($z_g$) from an encoder of the GAT-VAE module with atleast one latent vector corresponding to a primer string ($z_s$) from the encoder of the SMILES-VAE module to form a combined latent vector ($z$).

13. The one or more non-transitory machine-readable information storage mediums of claim 11, wherein the conditional molecular generator is pretrained with training datasets of one or more active site graphs and one or more molecules.

14. The one or more non-transitory machine-readable information storage mediums of claim 11, wherein designing at least one small molecule for the target protein is based on applying one or more physio chemical properties and toxicity filters on each target protein specific to the molecule from the set of molecules to obtain a reduced set of target specific molecules.

15. The one or more non-transitory machine-readable information storage mediums of claim 11, wherein the set of molecules are associated with a binding affinity which is greater than or equal to the predefined threshold score.

**Patentansprüche**

1. Prozessorimplementiertes Verfahren (300) für strukturbasiertes Arzneimitteldesign unter Verwendung eines multimodalen Deep-Learning-Modells, umfassend:

Verarbeiten (302), über einen oder mehrere Hardwareprozessoren (104), einer Eingabe mit einem Zielprotein für Arzneimitteldesign unter Verwendung von:

(a) einem multimodalen Deep-Learning-Modell, wobei das multimodale Learning-Modell ein Graph-Attention-based Variational Auto-Encoder (GAT-VAE)-Modul umfasst,
(b) einem Simplified Molecular Input Line Entry System Based Variational Auto-Encoder (SMILES-VAE)-Modul,
(c) einem bedingten molekularen Generator, und
(d) einem Drug-Target Affinity (DTA)-Prädiktormodul;

Erhalten (304), über den einen oder die mehreren Hardwareprozessoren (104), unter Verwendung des GAT-VAE-Moduls von dem Zielprotein, eines latenten Vektors von mindestens einem von einem aktiven Stellengraph, der Schlüsselaminosäurereste umfasst, wobei das GAT-VAE-Modul vortrainiert ist, um Struktur und Typ von Interaktionen von Aminosäuren zu lernen, die die aktiven Stellenreste des Zielproteins auskleiden;
Erhalten (306), über den einen oder die mehreren Hardwareprozessoren (104), unter Verwendung des SMILES-VAE-Moduls, mindestens eines latenten Vektors von dem Zielprotein, wobei das SMILES-VAE-Modul vortrainiert ist, um die Grammatik von kleinen Molekülen zu lernen;
Verketten (308), über den einen oder die mehreren Hardwareprozessoren (104), unter Verwendung des bedingten molekularen Generators, mindestens eines latenten Vektors des aktiven Stellengraphen des GAT-VAE-Moduls mit dem mindestens einen latenten Vektor des SMILES-VAE-Moduls, um einen Satz von Molekülen zu erzeugen, die für das Zielprotein spezifisch sind;
iteratives Durchführen (310), über den einen oder die mehreren Hardwareprozessoren (104), durch ein Reinforcement Learning (RL)-Framework an dem verketteten latenten Vektor, um mindestens ein Molekül unter Verwendung des Drug-Target Affinity (DTA)-Prädiktormoduls zu optimieren, um einen Affinitätswert für den Satz von Molekülen zu dem Zielprotein vorherzusagen, wobei das DTA-Prädiktormodul unter Verwendung eines Arzneimittelproteindatensatzes vortrainiert ist; und
Entwerfen (312), über den einen oder die mehreren Hardwareprozessoren (104), unter Verwendung des bedingten Molekülgenerators, mindestens eines optimierten Moleküls mit einer Affinität des Zielproteins, die größer als eine vordefinierte Schwellenwertbewertung ist.

2. Prozessorimplementiertes Verfahren (300) nach Anspruch 1, wobei der bedingte molekulare Generator mindestens einen latenten Vektor des eingegebenen aktiven Stellengraphen ($z_g$) von einem Codierer des GAT-VAE-Moduls mit mindestens einem latenten Vektor, der einer Primerkette ($z_s$) von dem Codierer des SMILES-VAE-Moduls entspricht, verkettet, um einen kombinierten latenten Vektor (z) zu bilden.

3. Prozessorimplementiertes Verfahren (300) nach Anspruch 1, wobei der bedingte molekulare Generator mit Trainingsdatensätzen von einem oder mehreren aktiven Stellengraphen und einem oder mehreren Molekülen vortrainiert ist.

4. Prozessorimplementiertes Verfahren (300) nach Anspruch 1, wobei das Entwerfen mindestens eines kleinen Moleküls für das Zielprotein auf dem Anwenden eines oder mehrerer physiochemischer Eigenschaften und Toxizitätsfilter auf jedes Zielprotein basiert, das für das Molekül aus dem Satz von Molekülen spezifisch ist, um einen reduzierten Satz von zielspezifischen Molekülen zu erhalten.

5. Prozessorimplementiertes Verfahren (300) nach Anspruch 1, wobei der Satz von Molekülen mit einer Bindungsaffinität assoziiert ist, die größer als oder gleich der vordefinierten Schwellenwertbewertung ist.

6. System (100) für strukturbasiertes Arzneimitteldesign unter Verwendung eines multimodalen Deep-Learning-Modells, umfassend:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Verarbeiten einer Eingabe mit einem Zielprotein für Arzneimitteldesign unter Verwendung von:

(a) einem multimodalen Deep-Learning-Modell, wobei das multimodale Learning-Modell ein Graph-Attention-based Variational Auto-Encoder (GAT-VAE)-Modul umfasst,
(b) einem Simplified Molecular Input Line Entry System Based Variational Auto-Encoder (SMILES-VAE)-Modul,
(c) einem bedingten molekularen Generator, und
(d) einem Drug-Target Affinity (DTA)-Prädiktormodul;

Erhalten, unter Verwendung des GAT-VAE-Moduls von dem Zielprotein, eines latenten Vektors von mindestens einem von einem aktiven Stellengraph, der Schlüsselaminosäurereste umfasst, wobei das GAT-VAE-Modul vortrainiert ist, um Struktur und Typ von Interaktionen von Aminosäuren zu lernen, die die aktiven Stellenreste des Zielproteins auskleiden;
Erhalten, unter Verwendung des SMILES-VAE-Moduls, mindestens eines latenten Vektors von dem Zielprotein, wobei das SMILES-VAE-Modul vortrainiert ist, um die Grammatik von kleinen Molekülen zu lernen;
Verketten, unter Verwendung des bedingten molekularen Generators, mindestens eines latenten Vektors des aktiven Stellengraphen des GAT-VAE-Moduls mit dem mindestens einen latenten Vektor des SMILES-VAE-Moduls, um einen Satz von Molekülen zu erzeugen, die für das Zielprotein spezifisch sind;
iteratives Durchführen, durch ein Reinforcement Learning (RL)-Framework an dem verketteten latenten Vektor, um mindestens ein Molekül unter Verwendung des Drug-Target Affinity (DTA)-Prädiktormoduls zu optimieren, um einen Affinitätswert für den Satz von Molekülen zu dem Zielprotein vorherzusagen, wobei das DTA-Prädiktormodul unter Verwendung eines Arzneimittelproteindatensatzes vortrainiert ist; und
Entwerfen, unter Verwendung des bedingten Molekülgenerators, mindestens eines optimierten Moleküls mit einer Affinität des Zielproteins, die größer als eine vordefinierte Schwellenwertbewertung ist.

7. System (100) nach Anspruch 6, wobei der bedingte molekulare Generator mindestens einen latenten Vektor des eingegebenen aktiven Stellengraphen ($z_g$) von einem Codierer des GAT-VAE-Moduls mit mindestens einem latenten Vektor, der einer Primerkette ($z_s$) von dem Codierer des SMILES-VAE-Moduls entspricht, verkettet, um einen kombinierten latenten Vektor ($z$) zu bilden.

8. System (100) nach Anspruch 6, wobei der bedingte molekulare Generator mit Trainingsdatensätzen von einem oder mehreren aktiven Stellengraphen und einem oder mehreren Molekülen vortrainiert ist.

9. System (100) nach Anspruch 6, wobei das Entwerfen mindestens eines kleinen Moleküls für das Zielprotein auf dem Anwenden eines oder mehrerer physiochemischer Eigenschaften und Toxizitätsfilter auf jedes Zielprotein basiert, das für das Molekül aus dem Satz von Molekülen spezifisch ist, um einen reduzierten Satz von zielspezifischen Molekülen zu erhalten.

10. System (300) nach Anspruch 6, wobei der Satz von Molekülen mit einer Bindungsaffinität assoziiert ist, die größer als oder gleich der vordefinierten Schwellenwertbewertung ist.

11. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:

Verarbeiten einer Eingabe mit einem Zielprotein für Arzneimitteldesign unter Verwendung von:

(a) einem multimodalen Deep-Learning-Modell, das ein Graph-Attention-based Variational Auto-Encoder (GAT-VAE)-Modul umfasst,
(b) einem Simplified Molecular Input Line Entry System Based Variational Auto-Encoder (SMILES-VAE)-Modul,
(c) einem bedingten molekularen Generator, und
(d) einem Drug-Target Affinity (DTA)-Prädiktormodul;

Erhalten, unter Verwendung des GAT-VAE-Moduls von dem Zielprotein, eines latenten Vektors von mindestens einem von einem aktiven Stellengraph, der Schlüsselaminosäurereste umfasst, wobei das GAT-VAE-Modul vortrainiert ist, um Struktur und Typ von Interaktionen von Aminosäuren zu lernen, die die aktiven Stellenreste des Zielproteins auskleiden;
Erhalten, unter Verwendung des SMILES-VAE-Moduls, mindestens eines latenten Vektors von dem Zielprotein, wobei das SMILES-VAE-Modul vortrainiert ist, um die Grammatik von kleinen Molekülen zu lernen;
Verketten, unter Verwendung des bedingten molekularen Generators, mindestens eines latenten Vektors des aktiven Stellengraphen des GAT-VAE-Moduls mit dem mindestens einen latenten Vektor des SMILES-VAE-Moduls, um einen Satz von Molekülen zu erzeugen, die für das Zielprotein spezifisch sind;

iteratives Durchführen, durch ein Reinforcement Learning (RL)-Framework an dem verketteten latenten Vektor, um mindestens ein Molekül unter Verwendung des Drug-Target Affinity (DTA)-Prädiktormoduls zu optimieren, um einen Affinitätswert für den Satz von Molekülen zu dem Zielprotein vorherzusagen, wobei das DTA-Prädiktormodul unter Verwendung eines Arzneimittelproteindatensatzes vortrainiert ist; und

Entwerfen, unter Verwendung des bedingten Molekülgenerators, mindestens eines optimierten Moleküls mit einer Affinität des Zielproteins, die größer als eine vordefinierte Schwellenwertbewertung ist.

12. Ein oder mehrere nichtflüchtige maschinenlesbare Informationsspeichermedien nach Anspruch 11, wobei der bedingte molekulare Generator mindestens einen latenten Vektor des eingegebenen aktiven Stellengraphen ($z_g$) von einem Codierer des GAT-VAE-Moduls mit mindestens einem latenten Vektor, der einer Primerkette ($z_s$) von dem Codierer des SMILES-VAE-Moduls entspricht, verkettet, um einen kombinierten latenten Vektor (z) zu bilden.

13. Ein oder mehrere nichtflüchtige maschinenlesbare Informationsspeichermedien nach Anspruch 11, wobei der bedingte molekulare Generator mit Trainingsdatensätzen von einem oder mehreren aktiven Stellengraphen und einem oder mehreren Molekülen vortrainiert ist.

14. Ein oder mehrere nichtflüchtige maschinenlesbare Informationsspeichermedien nach Anspruch 11, wobei das Entwerfen mindestens eines kleinen Moleküls für das Zielprotein auf dem Anwenden eines oder mehrerer physiochemischer Eigenschaften und Toxizitätsfilter auf jedes Zielprotein basiert, das für das Molekül aus dem Satz von Molekülen spezifisch ist, um einen reduzierten Satz von zielspezifischen Molekülen zu erhalten.

15. Ein oder mehrere nichtflüchtige maschinenlesbare Informationsspeichermedien nach Anspruch 11, wobei der Satz von Molekülen mit einer Bindungsaffinität assoziiert ist, die größer als oder gleich der vordefinierten Schwellenwertbewertung ist.

## Revendications

1. Procédé mis en œuvre par processeur (300) pour la conception de médicaments basée sur la structure en utilisant un modèle d'apprentissage profond multimodal, comprenant :
le traitement (302), via un ou plusieurs processeurs matériels (104), d'une entrée ayant une protéine cible pour la conception de médicaments en utilisant :

   (a) un modèle d'apprentissage profond multimodal, dans lequel le modèle d'apprentissage multimodal comprend un module auto-codeur variationnel basé sur l'attention graphique (GAT-VAE),
   (b) un module auto-codeur variationnel basé sur le système d'entrée de ligne d'entrée moléculaire simplifié (SMILES-VAE),
   (c) un générateur moléculaire conditionnel, et
   (d) un module prédicteur d'affinité médicament-cible (DTA) ;

      l'obtention (304), via les un ou plusieurs processeurs matériels (104), en utilisant le module GAT-VAE à partir de la protéine cible, d'un vecteur latent d'au moins l'un d'un graphique de site actif comprenant des résidus d'acides aminés clés, dans lequel le module GAT-VAE est préentraîné pour apprendre la structure et le type d'interactions à partir d'acides aminés recouvrant les résidus de site actif de la protéine cible ;
      l'obtention (306), via les un ou plusieurs processeurs matériels (104), en utilisant le module SMILES-VAE, d'au moins un vecteur latent à partir de la protéine cible, dans lequel le module SMILES-VAE est préentraîné pour apprendre la grammaire de petites molécules ;
      la concaténation (308), via les un ou plusieurs processeurs matériels (104), en utilisant le générateur moléculaire conditionnel, d'au moins un vecteur latent du graphique de site actif du module GAT-VAE avec l'au moins un vecteur latent du module SMILES-VAE pour générer un ensemble de molécules spécifiques à la protéine cible ;
      la réalisation itérative (310), via les un ou plusieurs processeurs matériels (104), par un cadre d'apprentissage de renforcement (RL) sur le vecteur latent concaténé pour optimiser au moins une molécule en utilisant le module prédicteur d'affinité médicament-cible (DTA) pour prédire une valeur d'affinité pour l'ensemble de molécules vers la protéine cible, dans lequel le module prédicteur DTA est préentraîné en utilisant un ensemble de données de protéine de médicament ; et

la conception (312), via les un ou plusieurs processeurs matériels (104), en utilisant le générateur moléculaire conditionnel, d'au moins une molécule optimisée avec une affinité de la protéine cible supérieure à un score de seuil prédéfini.

2. Procédé mis en œuvre par processeur (300) selon la revendication 1, dans lequel le générateur moléculaire conditionnel concatène au moins un vecteur latent du graphique de site actif d'entrée ($z_g$) à partir d'un codeur du module GAT-VAE avec au moins un vecteur latent correspondant à une chaîne d'amorces ($z_s$) à partir du codeur du module SMILES-VAE pour former un vecteur latent combiné (z).

3. Procédé mis en œuvre par processeur (300) selon la revendication 1, dans lequel le générateur moléculaire conditionnel est préentraîné avec des ensembles de données d'apprentissage d'un ou plusieurs graphiques de site actif et d'une ou plusieurs molécules.

4. Procédé mis en œuvre par processeur (300) selon la revendication 1, dans lequel la conception d'au moins une petite molécule pour la protéine cible est basée sur l'application d'une ou plusieurs propriétés physiochimiques et de filtres de toxicité sur chaque protéine cible spécifique à la molécule de l'ensemble de molécules pour obtenir un ensemble réduit de molécules spécifiques à la cible.

5. Procédé mis en œuvre par processeur (300) selon la revendication 1, dans lequel l'ensemble de molécules est associé à une affinité de liaison qui est supérieure ou égale au score de seuil prédéfini.

6. Système (100) pour la conception de médicaments basée sur la structure en utilisant un modèle d'apprentissage profond multimodal, comprenant :

une mémoire (102) stockant des instructions ;
une ou plusieurs interfaces de communication (106) ; et
un ou plusieurs processeurs matériels (104) couplés à la mémoire (102) via les une ou plusieurs interfaces de communication (106), dans lequel les un ou plusieurs processeurs matériels (104) sont configurés par les instructions pour :

traiter une entrée ayant une protéine cible pour la conception de médicaments en utilisant :

(a) un modèle d'apprentissage profond multimodal, dans lequel le modèle d'apprentissage multimodal comprend un module auto-codeur variationnel basé sur l'attention graphique (GAT-VAE),
(b) un module auto-codeur variationnel basé sur le système d'entrée de ligne d'entrée moléculaire simplifié (SMILES-VAE),
(c) un générateur moléculaire conditionnel, et
(d) un module prédicteur d'affinité médicament-cible (DTA) ;

obtenir, en utilisant le module GAT-VAE à partir de la protéine cible, un vecteur latent d'au moins l'un d'un graphique de site actif comprenant des résidus d'acides aminés clés, dans lequel le module GAT-VAE est préentraîné pour apprendre la structure et le type d'interactions à partir d'acides aminés recouvrant les résidus de site actif de la protéine cible ;
obtenir, en utilisant le module SMILES-VAE, au moins un vecteur latent à partir de la protéine cible, dans lequel le module SMILES-VAE est préentraîné pour apprendre la grammaire de petites molécules ;
concaténer, en utilisant le générateur moléculaire conditionnel, au moins un vecteur latent du graphique de site actif du module GAT-VAE avec l'au moins un vecteur latent du module SMILES-VAE pour générer un ensemble de molécules spécifiques à la protéine cible ;
réaliser itérativement, par un cadre d'apprentissage de renforcement (RL) sur le vecteur latent concaténé pour optimiser au moins une molécule en utilisant le module prédicteur d'affinité médicament-cible (DTA) pour prédire une valeur d'affinité pour l'ensemble de molécules vers la protéine cible, dans lequel le module prédicteur DTA est préentraîné en utilisant un ensemble de données de protéine de médicament ; et
concevoir, en utilisant le générateur moléculaire conditionnel, au moins une molécule optimisée avec une affinité de la protéine cible supérieure à un score de seuil prédéfini.

7. Système (100) selon la revendication 6, dans lequel le générateur moléculaire conditionnel concatène au moins un vecteur latent du graphique de site actif d'entrée ($z_g$) à partir d'un codeur du module GAT-VAE avec au moins un vecteur latent correspondant à une chaîne d'amorces ($z_s$) à partir du codeur du module SMILES-VAE pour former un vecteur latent combiné (z).

8. Système (100) selon la revendication 6, dans lequel le générateur moléculaire conditionnel est préentraîné avec des ensembles de données d'apprentissage d'un ou plusieurs graphiques de site actif et d'une ou plusieurs molécules.

9. Système (100) selon la revendication 6, dans lequel la conception d'au moins une petite molécule pour la protéine cible est basée sur l'application d'une ou plusieurs propriétés physiochimiques et de filtres de toxicité sur chaque protéine cible spécifique à la molécule de l'ensemble de molécules pour obtenir un ensemble réduit de molécules spécifiques à la cible.

10. Système (300) selon la revendication 6, dans lequel l'ensemble de molécules est associé à une affinité de liaison qui est supérieure ou égale au score de seuil prédéfini.

11. Un ou plusieurs supports de stockage d'informations lisibles par machine non transitoires comprenant une ou plusieurs instructions qui, lorsqu'elles sont exécutées par un ou plusieurs processeurs matériels, amènent :

le traitement d'une entrée ayant une protéine cible pour la conception de médicaments en utilisant :

(a) un modèle d'apprentissage profond multimodal comprenant un module auto-codeur variationnel basé sur l'attention graphique (GAT-VAE),
(b) un module auto-codeur variationnel basé sur le système d'entrée de ligne d'entrée moléculaire simplifié (SMILES-VAE),
(c) un générateur moléculaire conditionnel, et
(d) un module prédicteur d'affinité médicament-cible (DTA) ;

l'obtention, en utilisant le module GAT-VAE à partir de la protéine cible, d'un vecteur latent d'au moins l'un d'un graphique de site actif comprenant des résidus d'acides aminés clés, dans lequel le module GAT-VAE est préentraîné pour apprendre la structure et le type d'interactions à partir d'acides aminés recouvrant les résidus de site actif de la protéine cible ;
l'obtention, en utilisant le module SMILES-VAE, d'au moins un vecteur latent à partir de la protéine cible, dans lequel le module SMILES-VAE est préentraîné pour apprendre la grammaire de petites molécules ;
la concaténation, en utilisant le générateur moléculaire conditionnel, d'au moins un vecteur latent du graphique de site actif du module GAT-VAE avec l'au moins un vecteur latent du module SMILES-VAE pour générer un ensemble de molécules spécifiques à la protéine cible ;
la réalisation itérative, par un cadre d'apprentissage de renforcement (RL) sur le vecteur latent concaténé pour optimiser au moins une molécule en utilisant le module prédicteur d'affinité médicament-cible (DTA) pour prédire une valeur d'affinité pour l'ensemble de molécules vers la protéine cible, dans lequel le module prédicteur DTA est préentraîné en utilisant un ensemble de données de protéine de médicament ; et
la conception, en utilisant le générateur moléculaire conditionnel, d'au moins une molécule optimisée avec une affinité de la protéine cible supérieure à un score de seuil prédéfini.

12. Un ou plusieurs supports de stockage d'informations lisibles par machine non transitoires selon la revendication 11, dans lequel le générateur moléculaire conditionnel concatène au moins un vecteur latent du graphique de site actif d'entrée ($z_g$) à partir d'un codeur du module GAT-VAE avec au moins un vecteur latent correspondant à une chaîne d'amorces ($z_s$) à partir du codeur du module SMILES-VAE pour former un vecteur latent combiné (z).

13. Un ou plusieurs supports de stockage d'informations lisibles par machine non transitoires selon la revendication 11, dans lequel le générateur moléculaire conditionnel est préentraîné avec des ensembles de données d'apprentissage d'un ou plusieurs graphiques de site actif et d'une ou plusieurs molécules.

14. Un ou plusieurs supports de stockage d'informations lisibles par machine non transitoires selon la revendication 11, dans lequel la conception d'au moins une petite molécule pour la protéine cible est basée sur l'application d'une ou plusieurs propriétés physiochimiques et de filtres de toxicité sur chaque protéine cible spécifique à la molécule de l'ensemble de molécules pour obtenir un ensemble réduit de molécules spécifiques à la cible.

15. Un ou plusieurs supports de stockage d'informations lisibles par machine non transitoires selon la revendication 11, dans lequel l'ensemble de molécules est associé à une affinité de liaison qui est supérieure ou égale au score de seuil prédéfini.

**System 100**

**Hardware Processor(s)  104**

**I/O Interface(s)  106**

**Memory 102**

**Modules  108**

**FIG. 1**

1

FIG. 2

300

process an input having a target protein for drug design by using atleast one of a multi-modal deep learning model comprising of a graph attention-based VAE (GAT-VAE) module, a simplified molecular input line entry system based variational auto-encoder (SMILES-VAE) module, a conditional molecular generator, and a drug-target affinity (DTA) predictor module

302

obtain by using the GAT-VAE module, from the target protein, a latent vector of at least one of active site graph comprising of key amino acid residues

304

obtain using the SMILES-VAE module, atleast one latent vector from the target protein, wherein the SMILES-VAE module is pretrained to learn the grammar of small molecules

306

A

FIG.3A

A

concatenate using the conditional molecular generator, the latent vector of active site graph of the GAT-VAE model with atleast one latent vector of the SMILES-VAE model to generate a set of molecules specific to the target protein — 308

iteratively perform by the reinforcement learning (RL) framework on the concatenated latent vectors to optimize at least one molecule by using the DTA predictor module to predict an affinity value for the set of molecules towards the target protein — 310

design, at least one optimized molecule with an affinity of the target protein greater than a pre-defined threshold score by using the conditional molecule generator — 312

**FIG.3B**

FIG.4

**FIG.5**

FIG.6

FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- PaccMann'RL: Designing anticancer drugs from transcriptomic data via reinforcement learning. **JANNIS BORN et al.** ARXIV.ORG. CORNELL UNIVERSITY LIBRARY, 29 August 2019